# EUROPEAN PATENT SPECIFICATION

(11) **EP 2 082 262 B1**
(45) Date of publication and mention of the grant of the patent: **12.12.2012**
(21) Application number: 07805443.4
(22) Date of filing: 04.10.2007
(51) Int. Cl.: G01S 15/89

(54) **3D ULTRASONIC COLOR FLOW IMAGING WITH GRAYSCALE INVERT**
3D-ULTRASCHALLFARBFLUSSBILDGEBUNG MIT GRAUSKALAUMKEHRUNG
IMAGERIE COULEUR À ULTRASONS EN TROIS DIMENSIONS AVEC INVERSION DE L'ÉCHELLE DES GRIS

(30) Priority: 13.10.2006 US 829353 P
(43) Date of publication of application: 29.07.2009
(73) Proprietor: Koninklijke Philips Electronics N.V., 5621 BA Eindhoven (NL)
(72) Inventor: SNYDER, Richard, A., Bothell, Washington 98041-3003 (US)
(74) Representative: Verweij, Petronella Danielle
(86) International application number: PCT/IB2007/054044
(87) International publication number: WO 2008/044173

(56) References cited:
- EP-A- 0 797 106
- WO-A-00/41003
- DE-A1- 19 819 800
- JP-A- 2001 061 839
- US-A- 6 117 080
- HASHIMOTO H ET AL: "Ultrasound 3-dimensional image processing using power Doppler image" ULTRASONICS SYMPOSIUM, 1995. PROCEEDINGS., 1995 IEEE SEATTLE, WA, USA 7-10 NOV. 1995, NEW YORK, NY, USA,IEEE, US, 7 November 1995 (1995-11-07), pages 1423-1426, XP010157377 ISBN: 0-7803-2940-6

## Description

This invention relates to medical diagnostic ultrasound systems and, in particular, to ultrasound systems which perform three dimensional (3D) color flow imaging.

It is common practice in ultrasonic diagnostic imaging to visualize the interior surface of structures of a human body, such as the ventricles of the fetal heart, using 3D volume rendered imaging. Using a 3D data acquisition technique known as Spatial Temporal Image Correlation (STIC), the dynamics of the fetal heart can be captured as a series of volumes, each captured as a Cineloop® of consecutive image frames. It is also common practice to capture 3D volumes of color flow data and form a 2D image projection of that data volume using various 3D volume rendering techniques. The 3D color flow data can then be viewed as a cross-sectional plane, or as a 3D image using various 3D rendering methods. Furthermore, the 3D color flow data can be acquired using the STIC technique to capture the hemodynamics of the fetal heart.

Another known technique for fetal imaging is known as "invert imaging." In invert imaging, the conventional grayscale range which generally shows structures in the body which return strong echoes as brightly displayed and anechoic structures such as blood which return little echo energy as dark, in an inverted grayscale range. This reversal of the grayscale range results in the blood inside of vessels as shown brightly lighted with the tissue of the surrounding vessels dimly displayed or invisible, thereby highlighting blood pools and vessel blood flow. See, for instance, US Pat. 6,117,080 (Schartz), which applies this technique in the detection of fluid-filled cysts.

The use of the fetal STIC technique in combination with invert imaging has provided clinicians with new insight into the structure of the fetal heart. This is important because the clinician is often presented with the problem of assessing the fetal heart for normal or abnormal formation and function. The conventional 3D invert image, by itself, does not provide any information about the hemodynamics of the heart. For that, the clinician must turn instead to another image, typically a cross-sectional color flow slice through the volume to assess blood flow velocity, and mentally correlate the flow of the cross-sectional color flow image with the 3D grayscale image formed with the invert technique. Accordingly it would be desirable to provide the clinician with a single imaging technique which simultaneously provides the vascular flow path information of an inverted image and the flow velocity information of the color flow slice.

US patent n° 5954653 discloses a method to combine inverted grayscale and flow data in a composite image.

In accordance with the principles of the present invention, 3D grayscale data is combined with 3D color flow data as specified in claim 1 or 7. In an illustrated example of the invention, the 3D projection of the surface rendering of the color volume data is combined with the 3D projection of the surface rendering of the "inverted" grayscale volume data to produce an image of the two together. In this example the process of combining the two 3D projections compares the value of the grayscale projection data at a given pixel location with the value of the color projection data at the same pixel location. If the grayscale value is below a certain threshold, only the grayscale value is used for the image pixel in the combined image. If the grayscale value is above that threshold, the grayscale value is added to the color value and that new value is used for the image pixel in the combined image. Other, image data algorithms can also be used to enhance features of the image.

In the drawings:
FIGURE 1 illustrates in block diagram form an ultrasonic diagnostic imaging system constructed in accordance with the principles of the present invention.
FIGURE 2 illustrates a flowchart of an example of a method of the present invention.
FIGURES 3 and 4 illustrate two ultrasound images produced in accordance with the principles of the present invention and captured during diastole and systole, respectively.

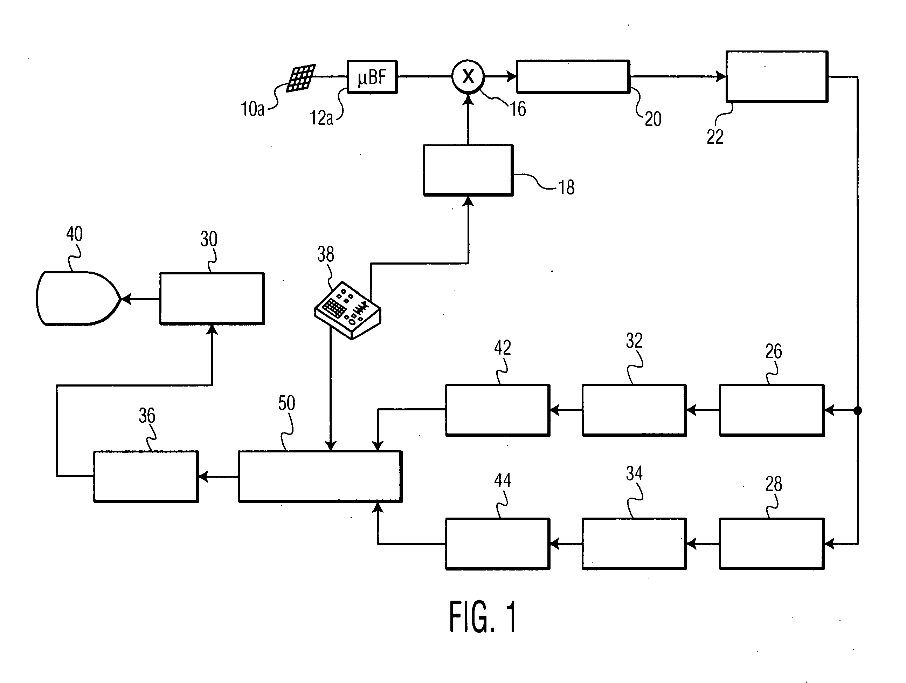

Referring first to FIGURE 1, an ultrasound system constructed in accordance with the principles of the present invention is shown in block diagram form. A transducer array 10a is provided for transmitting ultrasonic waves and receiving echo signals. In this example the array shown is a two dimensional array of transducer elements capable of providing 3D image information, although an implementation of the present invention may also use a swept one dimensional array of transducer elements which produces 2D (planar) images from a volumetric region. The transducer array is coupled to a microbeamformer 12a which controls transmission and reception of signals by the array elements. The microbeamformer is also capable of at least partial beamforming of the signals received by groups or "patches" of transducer elements as described in US Pats. 5,997,479 (Savord et al.), 6,013,032 (Savord), and 6,623,432 (Powers et al.) The microbeamformer 12a is coupled to a transmit/receive (T/R) switch 16 which switches between transmission and reception and protects the main beamformer 20 from high energy transmit signals. The transmission of ultrasonic beams from the transducer array 10a is under control of a transmit controller 18 coupled to the T/R switch, which receives input from the user's operation of the user interface or control panel 38.

The partially beamformed signals produced by the microbeamformer 12a are coupled to a main beamformer 20 where partially beamformed signals from the individual patches of elements are combined into a fully beamformed signal. For example, the main beamformer 20 may have 128 channels, each of which receives a partially beamformed signal from a patch of 12 transducer elements. In this way the signals received by over 1500 transducer elements of a two dimensional array can contribute efficiently to a single beamformed signal. The beamformed signals are coupled to a signal processor 22 where they may undergo additional enhancement such as speckle removal, signal compounding, harmonic separation, filtering, multiline interpolation and processing, and noise elimination.

The processed signals are coupled to a B mode processor 26 and a Doppler processor 28. The B mode processor 26 employs amplitude detection for the imaging of structures in the body such as muscle, tissue, and blood cells. B mode images of structure of the body may be formed in either the harmonic mode or the fundamental mode. Tissues in the body and microbubbles both return both types of signals and the harmonic returns of microbubbles enable microbubbles to be clearly segmented in an image in most applications. The Doppler processor processes temporally distinct signals from tissue and blood flow for the detection of motion of substances in the image field including blood cells, tissue, and microbubbles. The Doppler processor operates on ensembles of temporally distinct samples from each location in the volume being imaged to produce an estimate of Doppler power, velocity, acceleration, or variance at each location in the volumetric region, the same volumetric region which is the source of the B mode signals. Different transmit signals may be used for B mode and Doppler returns or the same signals may be used by both processors as described in US Pat. 6,139,501 (Roundhill et al.) The Doppler processor 28 can operate on the I,Q quadrature data typically produced by the signal processor 22 and the B mode processor 26 can operate on the same data in the form of (I² + Q²)^{1/2}. The structural B mode signals are coupled to a grayscale mapping processor 32 which converts the B mode signals to a range of grayscale values. The flow signals from the Doppler processor 28 are coupled to a color mapping processor 34 which similarly converts the flow signals to a range of color values. When the flow signals are velocity-related signals for color flow imaging the range of color values corresponds to a range of flow velocities, for instance. Other Doppler modes such as power Doppler, acceleration, and variance may be used if desired. The mapping processors may implement grayscale and color ranges selected by the user and may be constructed as lookup tables. When the ultrasound system of FIGURE 1 is producing an image in accordance with the present invention the grayscale map is inverted from its conventional scale, with stronger B mode signals being converted to darker grayscale values and weaker B mode signals converted to brighter grayscale values. This will cause the stronger echoes from the tissue of blood vessel walls to be displayed less brightly than the weaker anechoic echo returns from blood flow within the vessel, for instance.

In accordance with the present invention the grayscale 3D data set and the 3D flow data set are each volume rendered to form a 2D display of each 3D volume of data by volume renderers 42 and 44, respectively. In practice one volume renderer may be used which is multiplexed to render one data set and then the other. Volume rendering is well known and is described in US Pat. 5,720,291 (Schwartz), for instance. See also US Pat. 6,530,885 (Entrekin et al.) Volume rendering can produce projections of a 3D volume from a series of different look directions and the user can then sequence through the look directions to view the 3D volume from different perspectives, a display format known as kinetic parallax. The volume renderers 42 and 44 can operate on image data in either rectilinear or polar coordinates as described in US Pat. 6,723,050 (Dow et al.)

In further accord with the present invention the volume rendered, "inverted" grayscale data produced by the volume renderer 42 and the volume rendered flow data produced by the volume renderer 44 are blended together by a pixel comparator and 3D image merge processor 50, which allows the flow path of blood vessels and heart chambers to be visualized together with the fluid motion characteristics of the flow path or chamber. One way to do this is to compare the B mode and flow data at each point in the image with a threshold. For instance, according to the invention one technique is to compare the grayscale value at an image location with a threshold and if the grayscale value is below the threshold, the grayscale value alone is used for display. But if the grayscale value exceeds the threshold, the grayscale and flow values are summed, resulting in a display pixel at that location which has been tinted with the flow value such as flow velocity. In addition, the flow value can be first compared with a threshold and used for display if it exceeds the threshold. If the flow value does not exceed the threshold the grayscale value is used for display at that location. In either case, the thresholds may be controlled and set by the user from the control panel 38, if desired. The result of this processing is that a single volume rendered (3D) image is used which contains characteristics of both of the initial volume renderings. The resultant merged image is coupled to a Cineloop buffer 36 where it may be displayed with a sequence of similarly processed imaged to visualize the hemodymanics of the heart, for instance. The images to be displayed are coupled to an image processor 30 for display on an image display 40.

To recap this processing, the formation of the combined grayscale and color 3D image projections is realized in a series of processing steps. These steps involve first the formation of the individual grayscale and color data projections, and then the final step of combining or compositing them into a single image for display. The formation of the grayscale image projection is realized through the rendering of grayscale data using an inverted grayscale map from the conventional map, where the first step in the process is to reverse, or invert the intensity of the individual voxels of the volume of data to be visualized. This inversion is such that it causes bright voxels to become dark, and dark voxels to become bright. Subsequent to this inversion step, a conventional 3D rendering technique such as the ray-cast method previously described is used to create a 2D image which is a projection of the 3D data as observed from a given viewpoint. Preferably, this rendering method is configured to find and show "surfaces" in the data, surfaces meaning generally the transition from low intensity (dark) voxels to higher intensity (bright) voxels as seen by a "ray" traveling from the observer into the volume of data. Because of the first step of inverting the intensities of the voxel data, the surfaces found and displayed during the rendering process are the equivalent to the interior surfaces of tissue which oppose anechoic, or normally dark regions present in the volume data. If the volume data includes a fetal heart, for example, the surfaces found and displayed would correspond to the interior surfaces of the ventricles of the heart and associated blood vessels connecting to it. The resulting 2D image is composed of MXN pixels, with each pixel consisting of a red (R), green (G), and blue (B) value. Typically these RGB values will all be equal to one another so that the displayed pixel will have a neutral (grey) color, although other tints can be used.

The formation of a color flow image projection is realized through conventional 3D rendering methods, such as the ray-cast method, to find and display the surface of targets that were previously detected by the ultrasound system to have a Doppler shift associated with them (moving blood cells) within the volume data, as viewed from a particular viewpoint. The resulting 2D image is composed of MXN pixels, each pixel consisting of a red (R), blue (B), and green (G) value. The RGB values of the pixels will be such that the resulting color displayed at each pixel location will correspond to the direction and velocity of the blood cells at that location as determined by a color map that relates velocity and direction as determined by the Doppler shift of the received echoes to displayed color.

The combined color and grayscale 2D projections of the 3D data created above are combined, or composited, in a non-linear fashion where each pixel of the combined image is formed as a combination of the pixel (voxel) data from the corresponding location in the grayscale and color images. There are several ways to combine the pixel data from the two images. According to the invention, one way is to first compare the grayscale pixel value to a selected threshold. If the pixel value falls below an adjustable threshold, only that RGB pixel value from the grayscale image is used for the combined image pixel, that is, only the grayscale data is displayed. On the other hand, if the pixel value from the grayscale data exceeds the adjustable threshold, then that pixel value is summed with the pixel value of the color image (R, G, and B summed individually) at that location, resulting in a grayscale pixel value that has been tinted with the corresponding velocity found at that same location. If desired, a range check can be performed to clamp the summed pixel value to full-brightness. This compositing method is opposite to the conventional process where color data is only displayed in the absence of grayscale data.

Another way to composite the grayscale and color images is to use an additional adjustable threshold for the values of the color pixel data such that the velocity of the color data is also taken into consideration. In this case, it may be desired to show the color pixel data regardless of the grayscale data if the velocity of the color data exceeds the adjustable threshold.

Yet another factor that can influence the compositing process is to consider the location in the volume from which the grayscale surface and the color surface portrayed by the grayscale and color image pixels originated. When using the ray-cast method of volume rendering, the final result is a two dimensional projection in which the depth dimension is normally lost. However, the depth location in the original volume data set from which the pixel originated can be determined by keeping track of the distance along the ray where the surface was found for each of the grayscale and color surfaces. Then, in addition to comparing the grayscale and color pixel values as part of the compositing process, the depths along the ray where each pixel was encountered can also be considered. For instance, when the grayscale pixel value in the grayscale rendering originated from a shallower depth than the corresponding color pixel value in the color flow rendering, the two values should not be merged as they relate to different locations in the volumetric region. In such case, if the grayscale value exceeds the specified brightness threshold, but the depth along the ray was different between the color pixel and grayscale pixel by some adjustable threshold, only the grayscale pixel value alone will be used for the combined image pixel. This additional factor prevents the merging of rendered data which does not spatially correlate and thus should not be combined.

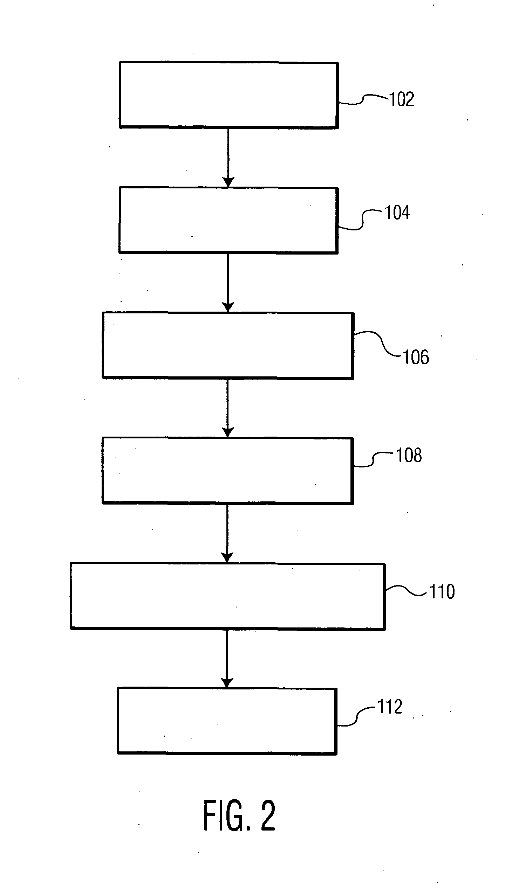

A simplified flowchart of a process of the present invention is shown in FIGURE 2. At 102 a 3D grayscale data set is acquired. AT 104 a 3D color flow data set is acquired. At 106 the grayscale data set is converted or mapped to an inverted grayscale map or range of values. At 108 the inverted 3D grayscale data set and the 3D color flow data set are volume rendered. At 110 the color flow values and grayscale values are combined or composited into a single 3D volume rendered image containing the characteristics of both data sets. A sequence of such images are then displayed in real time at 112.

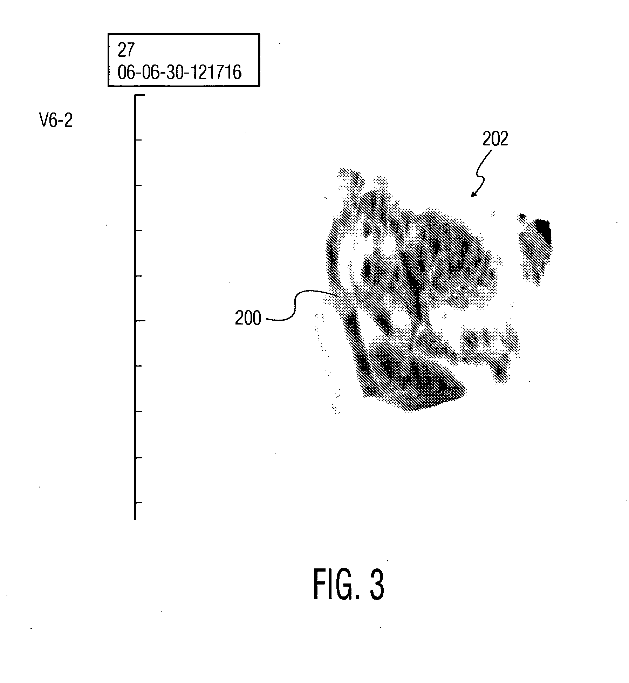

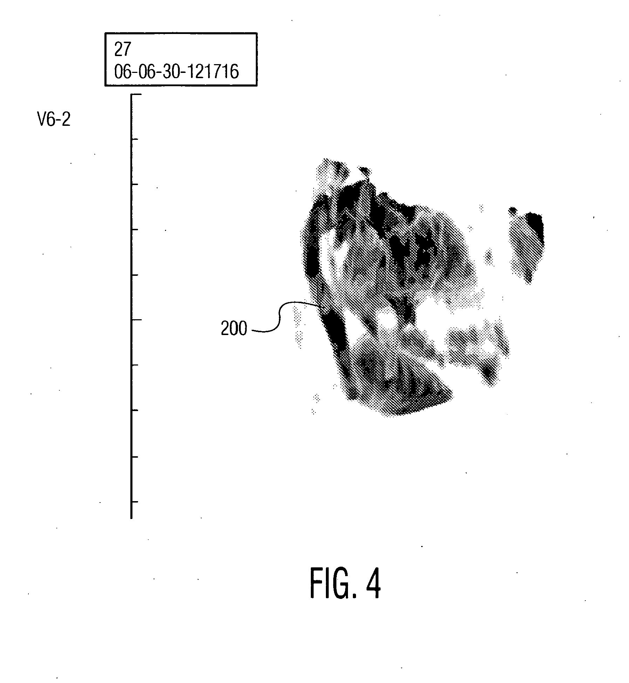

FIGURES 3 at 4 show two ultrasound images of a fetal heart, the first one acquired at diastole and the second one acquired at systole. These images were acquired by an operating implementation of the present invention and were displayed on the acquiring ultrasound system as color images shown against a black background. However, for purposes of patent illustration, this conventional display format has been reversed in the drawings so that the background is white and the flow paths and velocities are in a gray shading. The first characteristic that can be noted is that these images are not of the heart itself (myocardium), but of the blood within the heart. The myocardial tissue structure around these regions of blood flow have been caused to be translucent or disappear by the inversion of the grayscale map, producing an image of flow similar to that described in US Pat. 5,474,073 (Schwartz et al.) See also the aforementioned US Pat. 5,720,291 (Schwartz) which illustrates a technique for visualizing flow by rendering the Doppler data opaque while tissue is rendered translucent. In these images the flow paths are opaquely displayed so that the outer surfaces of the flow paths and blood pools are highlighted in the rendering, thus showing the flow of a continuous vessel as a continuous opaque "tube" of blood. In FIGURES 3 and 4 the clinician can note the locations and positions of the flow paths such as the aorta 200, examining their continuity and diagnosing the proper formation of the heart and vessels. During the diastolic phase of FIGURE 3 the left ventricle indicated at 202 is highly colored as blood fills this chamber of the heart. There is little or no color in the aorta 200 as the heart is filling during this phase. In the systolic phase of FIGURE 4 there is relatively less color in the left ventricle but the mitral valve region and the aorta 200 are brightly colored as the contraction of the heart forces blood out of the heart and into the aorta and surrounding vasculature. The clinician is thus able to make a diagnosis on the basis of one image which shows the full hemodynamic characteristics of the blood flow.

## Claims

1. An ultrasonic diagnostic imaging system for analyzing blood flow comprising:
a transducer array (10a) operable to transmit and receive ultrasonic signals over a volumetric region where blood flow is present;
a B mode processor (26) coupled to the transducer array which acts to produce B mode data of the volumetric region;
a Doppler processor (28) coupled to the transducer array which acts to produce flow data of the volumetric region;
a grayscale map (32) which produces an inverted mapping of the B-mode grayscale data which highlights anechoic return signals;
a volume renderer (42,44)coupled to the processors which acts to produce volume renderings of the inverted B mode grayscale data and the flow data;
an image merge processor (50)which combines the volume rendered flow data and inverted B mode grayscale data into a composite image,
a display (40) coupled to the image merge processor for displaying the composite image, **characterised in that** the image merge processor (50) further includes a pixel comparator adapted to compare the inverted B-mode grayscale value of a pixel at a given image location to a first threshold, and to assign the following value to said pixel:
- the sum of the inverted B-mode grayscale value and the flow value of said pixel, if the inverted B-mode grayscale value is above the first threshold,
- the same inverted B-mode grayscale value of said pixel if this value is below the first threshold.

2. The ultrasonic diagnostic imaging system of Claim 1, wherein the Doppler processor produces color flow data.

3. The ultrasonic diagnostic imaging system of Claim 2, further comprising a color map which operates to map the color flow data to a range of color values.

4. The ultrasonic diagnostic imaging system of Claim 1, wherein the image merge processor further includes a comparator which operates to compare flow data to a second threshold value.

5. The ultrasonic diagnostic imaging system of Claim 1, wherein the first threshold value further comprises a user adjustable threshold value.

6. The ultrasonic diagnostic imaging system of Claim 4, wherein the second threshold value further comprises a user adjustable threshold value.

7. A method for producing a 3D ultrasound image of tissue and flow comprising:
acquiring (102) a grayscale 3D data set;
acquiring (104) a color flow 3D data set;
mapping (106) the grayscale 3D data to produce an inverted mapping of the B-mode grayscale data thereby highlighting anechoic returns more greatly than strong echo returns;
volume rendering (108) the mapped inverted B-mode grayscale 3D data set and the color flow 3D data set;
combining the volume rendered inverted B-mode grayscale and color flow data on a spatial basis (110); and
displaying (112) a composite inverted B-mode grayscale and flow volume rendered image, **characterised by**
the step of combining further comprises comparing the inverted B-mode grayscale value of a pixel at a given image location to a first threshold, and assigning the following value to said pixel:
- the sum of the inverted B-mode grayscale value and the flow value of said pixel, if the inverted B-mode grayscale value is above the first threshold,
- the same inverted B-mode grayscale value of said pixel if this value is below the first threshold.

8. The method of Claim 7, wherein combining further comprises color flow data relating to a common spatial location to a threshold.

9. The method of Claim 8, wherein combining results in tinting the grayscale data values at locations in a 3D region with colors corresponding to the flow characteristics at those locations.

## Patentansprüche

1. Diagnostisches Ultraschall-Bildgebungssystem zur Analyse der Blutströmung, das Folgendes umfasst:
ein Wandler-Array (10a), das funktionsbereit ist, um Ultraschallsignale über eine Volumenregion zu senden und zu empfangen, in der eine Blutströmung vorhanden ist;
einen mit dem Wandler-Array gekoppelten B-Mode-Prozessor (26), der dazu dient, B-Mode-Daten der Volumenregion zu erzeugen;
einen mit dem Wandler-Array gekoppelten Doppler-Prozessor (28), der dazu dient, Strömungsdaten der Volumenregion zu erzeugen;
eine Grauskalenkarte (32), die eine invertierte Abbildung der B-Mode-Grauskalendaten erzeugt, welche echofreie Rücksignale hervorhebt;
eine mit den Prozessoren gekoppelte Volumendarstellungsvorrichtung (42, 44) (engl. volume renderer), die dazu dient, Volumendarstellungen der invertierten B-Mode-Grauskalendaten und der Strömungsdaten zu erzeugen;
einen Bildzusammenführungsprozessor (50), der die der Volumendarstellung unterzogenen Strömungsdaten und invertierten B-Mode-Grauskalendaten zu einem zusammengesetzten Bild kombiniert;
eine mit dem Bildzusammenführungsprozessor gekoppelte Anzeigevorrichtung (40) zum Anzeigen des zusammengesetzten Bildes, **dadurch gekennzeichnet, dass**
der Bildzusammenführungsprozessor (50) weiterhin einen Pixelkomparator umfasst, der vorgesehen ist, um den invertierten B-Mode-Grauskalenwert eines Pixels an einer gegebenen Bildposition mit einem ersten Schwellenwert zu vergleichen und dem genannten Pixel folgenden Wert zuzuweisen:
- die Summe aus invertiertem B-Mode-Grauskalenwert und Strömungswert des genannten Pixels, wenn der invertierte B-Mode-Grauskalenwert über dem ersten Schwellenwert liegt,
- den gleichen invertierten B-Mode-Grauskalenwert des genannten Pixels, wenn dieser Wert unter dem ersten Schwellenwert liegt.

2. Diagnostisches Ultraschall-Bildgebungssystem nach Anspruch 1, wobei der Doppler-Prozessor Farbströmungsdaten erzeugt.

3. Diagnostisches Ultraschall-Bildgebungssystem nach Anspruch 2, weiterhin mit einer Farbkarte, die dazu dient, die Farbströmungsdaten auf eine Reihe von Farbwerten abzubilden.

4. Diagnostisches Ultraschall-Bildgebungssystem nach Anspruch 1, wobei der Bildzusammenführungsprozessor weiterhin einen Komparator umfasst, der dazu dient, Strömungsdaten mit einem zweiten Schwellenwert zu vergleichen.

5. Diagnostisches Ultraschall-Bildgebungssystem nach Anspruch 1, wobei der erste Schwellenwert weiterhin einen vom Benutzer einstellbaren Schwellenwert umfasst.

6. Diagnostisches Ultraschall-Bildgebungssystem nach Anspruch 4, wobei der zweite Schwellenwert weiterhin einen vom Benutzer einstellbaren Schwellenwert umfasst.

7. Verfahren zum Erzeugen eines 3D-Ultraschallbildes von Gewebe und Strömung, das Folgendes umfasst:
Erfassen (102) eines Grauskalen-3D-Datensatzes;
Erfassen (104) eines Farbströmungs-3D-Datensatzes;
Abbilden (106) der Grauskalen-3D-Daten, um eine invertierte Abbildung der B-Mode-Grauskalendaten zu erzeugen und dadurch echofreie Rücksignale stärker hervorzuheben als starke Echosignale:
Volumendarstellung (108) des abgebildeten invertierten B-Mode-Grauskalen-3D-Datensatzes und des Farbströmungs-3D-Datensatzes;
Kombinieren der der Volumendarstellung unterzogenen invertierten B-Mode-Grauskalendaten und Farbströmungsdaten auf einer räumlichen Basis (110); und
Anzeigen (112) eines zusammengesetzten Bildes aus der Volumendarstellung unterzogenen invertierten B-Mode-Grauskalendaten und Strömungsdaten, **gekennzeichnet durch**
den Schritt des Kombinierens, welcher weiterhin das Vergleichen des invertierten B-Mode-Grauskalenwertes eines Pixels an einer gegebenen Bildposition mit einem ersten Schwellenwert und das Zuweisen des folgenden Wertes zu dem genannten Pixel umfasst:
- die Summe aus invertiertem B-Mode-Grauskalenwert und Strömungswert des genannten Pixels, wenn der invertierte B-Mode-Grauskalenwert über dem ersten Schwellenwert liegt,
- den gleichen invertierten B-Mode-Grauskalenwert des genannten Pixels, wenn dieser Wert unter dem ersten Schwellenwert liegt.

8. Verfahren nach Anspruch 7, wobei das Kombinieren weiterhin das Vergleichen von Farbströmungsdaten, die sich auf einen gemeinsamen räumlichen Ort beziehen, mit einem Schwellenwert umfasst.

9. Verfahren nach Anspruch 8, wobei das Kombinieren zu einer Tönung der Grauskalendatenwerte an Orten in einer 3D-Region mit Farben führt, die den Strömungseigenschaften an diesen Orten entsprechen.

## Revendications

1. Système d'imagerie diagnostique ultrasonique pour analyser un flux sanguin, comprenant :
un réseau de capteurs (10a) utilisable pour transmettre et recevoir des signaux ultrasoniques sur une région volumétrique où un flux sanguin est présent ;
un processeur mode B (26) couplé au réseau de capteurs qui sert à produire des données en mode B de la région volumétrique ;
un processeur Doppler (28) couplé au réseau de capteurs qui sert à produire des données de flux de la région volumétrique ;
une mappe à nuances de gris (32) qui produit un mappage inversé des données à nuances de gris en mode B qui met en évidence des signaux de retours anéchogènes;
un moteur de rendu volumique (42, 44) couplé aux processeurs qui sert à produire des rendus volumiques des données inversées à nuances de gris en mode B et des données de flux ;
un processeur de fusion d'image (50) qui combine les données de flux ayant subi un rendu volumique et les données inversées à nuances de gris en mode B dans une image composite,
un affichage (40) couplé au processeur de fusion d'image pour afficher l'image composite, **caractérisé en ce que**
le processeur de fusion d'image (50) comprend en outre un comparateur de pixel adapté pour comparer la valeur inversée à nuances de gris en mode B d'un pixel dans un emplacement d'image donné à un premier seuil, et à attribuer la valeur suivante audit pixel :
- la somme de la valeur inversée à nuances de gris en mode B et de la valeur de flux dudit pixel, si la valeur inversée à nuances de gris en mode B est supérieure au premier seuil,
- la même valeur inversée à nuances de gris en mode B dudit pixel si cette value est inférieure au premier seuil.

2. Système d'imagerie diagnostique ultrasonique selon la revendication 1, dans lequel le processeur Doppler produit des données de flux en couleur.

3. Système d'imagerie diagnostique ultrasonique selon la revendication 2, comprenant en outre une mappe de couleur qui fonctionne pour mapper les données de flux en couleur sur une plage de valeurs de couleur.

4. Système d'imagerie diagnostique ultrasonique selon la revendication 1, dans lequel le processeur de fusion d'image comprend en outre un comparateur qui fonctionne pour comparer des données de flux à une seconde valeur de seuil.

5. Système d'imagerie diagnostique ultrasonique selon la revendication 1, dans lequel la première valeur de seuil comprend en outre une valeur de seuil réglable par utilisateur.

6. Système d'imagerie diagnostique ultrasonique selon la revendication 4, dans lequel la seconde valeur de seuil comprend en outre une valeur de seuil réglable par utilisateur.

7. Procédé pour produire une image 3D par ultrasons de tissu et de flux, comprenant :
l'acquisition (102) d'un jeu de données 3D à nuances de gris ;
l'acquisition (104) d'un jeu de données 3D de flux en couleur ;
le mappage (106) des données 3D à nuances de gris pour produire un mappage inversé des données à nuances de gris en mode B, mettant ainsi plus en évidence des retours anéchogènes que des retours fortement échogènes ;
le rendu volumique (108) du jeu de données 3D mappées inversées à nuances de gris en mode B et du jeu de données 3D de flux en couleur ;
la combinaison des données de flux en couleur et inversées à nuances de gris en mode B ayant subi un rendu volumique sur une base spatiale (110) ; et
l'affichage (112) d'une image composite ayant subi un rendu volumique de données de flux et inversées à nuances de gris en mode B, **caractérisé en ce que**
l'étape de combinaison comprend en outre la comparaison de la valeur inversée à nuances de gris en mode B d'un pixel dans un emplacement d'image donné à un premier seuil, et l'attribution de la valeur suivante audit pixel :
- la somme de la valeur inversée à nuances de gris en mode B et la valeur de flux dudit pixel, si la valeur inversée à nuances de gris en mode B est supérieure au premier seuil,
- la même valeur inversée à nuances de gris en mode B dudit pixel si cette value est inférieure au premier seuil.

8. Procédé selon la revendication 7, dans lequel la combinaison comprend en outre la comparaison de données de flux en couleur concernant un emplacement spatial commun à un seuil.

9. Procédé selon la revendication 8, dans lequel la combinaison entraîne la coloration des valeurs de données à nuances de gris dans des emplacements dans une région 3D avec des couleurs correspondant aux caractéristiques de flux dans ces emplacements.
